# EUROPEAN PATENT SPECIFICATION

(11) **EP 2 457 506 B1**
(45) Date of publication and mention of the grant of the patent: **02.09.2015**
(21) Application number: 10192694.7
(22) Date of filing: 26.11.2010
(51) Int. Cl.: A61B 5/08, A61B 5/097, A61M 16/08, A61B 5/083

(54) **Liquid separation apparatus for removing a liquid from a respiratory gas and respiratory gas analyzing system**
Flüssigkeitstrennvorrichtung zum Entfernen einer Flüssigkeit aus einem Atemgas und Atemgas-Analysesystem
Appareil de séparation des liquides pour éliminer un liquide d'un gaz respiratoire et système d'analyse de gaz respiratoire

(43) Date of publication of application: 30.05.2012
(73) Proprietor: General Electric Company, Schenectady, NY 12345 (US)
(72) Inventor: Weckström, Kurt, 00031 GE, Helsinki (FI)
(74) Representative: Illingworth-Law, William Illingworth

(56) References cited:
- EP-A1- 2 233 167
- WO-A1-00/72941
- WO-A2-2005/118036
- US-A- 4 465 485

## Description

### BACKGROUND OF THE INVENTION

This disclosure relates generally to a liquid separation apparatus for removing a liquid from a respiratory gas comprising a housing having an input for delivering a sample withdrawn from the respiratory gas, a first output for discharging at least part of the sample of the respiratory gas and a first filter with a hydrophobic property for removing the liquid from the gas sample flowing through the input and first output of the housing. Also this disclosure relates to a respiratory gas analyzing system.

In anesthesia or in intensive care, the condition of a patient is often monitored e.g. by analyzing the gas exhaled by the patient for its content. For this reason a small portion of the respiratory gas may be diverted to a gas analyzer. This sample often carries along to the analyzer some water vapor, which condensates into droplets, and also some dust, water, mucus and blood. Such components carried along with the sample would have a detrimental effect on the gas analyzer and measuring result. This is why the dust and liquid components must be removed and collected from a gas sample upstream of the actual gas analyzer. In this so called the sidestream configuration, a liquid filtering device, also called a water trap, is located in series with the sampling line to separate water, mucus and other unwanted material from the gas sample. To improve the lung function and oxygenation of blood there is often a nebulizer connected to the inspiratory limb of the respiratory circuit. Except for humidity also different drugs can be administered deep in to the lung as tiny droplets. In many cases the surface tension of these drugs is lower than that of water. Sometimes a surfactant is intentionally added to the drug to be nebulized in order to improve its clinical efficiency. In the instructions for gas analyzers it is common practice to disconnect the sampling line during nebulizing.

Different kinds of liquid filtering devices at the input of a patient gas analyzer are well known in the art. In one device the upstream end of the sampling tube is provided with a tubular housing comprising a hydrophobic hollow fiber filter element. In order not to increase the response time of the gas analyzer the tubular housing must have small volume. It is possible that the device can handle a small amount of water but it is easily obstructed by mucus or blood. The device would then have to be replaced. This may happen quite often in critical care use and would decrease the cost-effectiveness of the device. Still worse, a reduced surface tension of a nebulized liquid sucked into the hydrophobic filter could wet the filter and decrease its water entry pressure to a level where water freely would enter the analyzer together with the drug.

In another type of water filtering devices the hydrophobic filter is located in the gas sampling adapter in the respiratory circuit. This solution effectively prevents water and mucus from entering the sampling line. In principle, it is an effective solution for critical care patients. On the other hand, an adapter is inconvenient, and even risky for the patient, to remove from the respiratory circuit for the nebulization procedure. This means that the hydrophobic filter membrane could become transmissible to water and allow it to enter the sampling line and gas analyzer.

Still another type of liquid filtering devices, a water trap with a liquid container, is also well-known. A passage, wherein a liquid component is separated from a gas flow, is divided into two sections by means of a gas permeable and water impermeable-material. Thus a sample picked up from the exhalation air of a patient is delivered into the first passage of a water separator, from which the water component along with a minor amount of gas is sucked away, usually by way of a liquid receiver or container. Most of the gas flow received in the first passage is sucked through the liquid impermeable hydrophobic material into the second passage and further to a gas analyzer. This hydrophobic filter material effectively prevents the passage of water to the gas analyzer. As earlier mentioned, permeability problems may occur if the liquid has reduced surface tension like many drugs used for nebulization. According to user instructions the sampling line should be disconnected during nebulization. In practice, this is inconvenient and can easily be overlooked. As a result the gas sensor and other internal parts of the gas analyzer may be contaminated.

US-4465485 discloses a suction canister assembly to collect body fluids. The assembly comprises a receptacle to collect liquid and a cover, which is sealably connected to the receptacle. The cover comprises a liquid passage opening communicating with a source of liquid, and a suction opening communicating with a source of vacuum to move the liquid from the source of liquid to the receptacle. The assembly also comprises a unitary shut-off valve/filter element through which the suction is applied. When the liquid is filling the receptacle, gas withdrawn from the receptacle is filtered in this valve/filter element to remove e.g. bacteria. When the liquid level of the receptacle contacts the valve/filter element, liquid collection is becoming slower or even no liquid can be drawn into the receptacle in which case the vacuum is closed off. The suction canister assembly needs to be disconnected and replaced with a new canister assembly.

### BREAF DESCRIPTION OF THE INVENTION

The above-mentioned shortcomings, disadvantages and problems are addressed herein which will be understood by reading and understanding the following specification.

In an embodiment, a liquid separation apparatus for removing a liquid from a respiratory gas includes a housing having an input for delivering a sample withdrawn from the respiratory gas, a first output for discharging at least part of the sample of the respiratory gas and a first filter with a hydrophobic property for removing the liquid from the gas sample flowing through the input and the first output of the housing. The first filter also having an oleophobic property to improve a removal of the liquid in case a surface tension of the liquid is lower than a surface tension of bodily fluids.

In another embodiment a respiratory gas analyzing system includes a gas analyzer for analyzing respiratory gases, which gas analyzer including a gas sensor for measuring at least one gas component, and a gas withdrawing apparatus for withdrawing respiratory gases to the gas sensor. The respiratory gas analyzing system also includes a liquid separation apparatus upstream the gas sensor comprising a housing having an input for delivering a sample withdrawn, a first output for discharging at least part of the sample of the respiratory gas to the gas sensor and a first filter with a hydrophobic property for removing the liquid from the gas sample flowing through the input of the housing. The first filter also having an oleophobic property to improve a removal of the liquid in case a surface tension of the liquid is lower than a surface tension of bodily fluids.

In yet another embodiment a liquid separation apparatus for removing a liquid from a respiratory gas includes a housing having an input for delivering a sample withdrawn from the respiratory gas, a first output for discharging at least part of the sample of the respiratory gas, and a first filter with a hydrophobic property for removing the liquid from the gas sample flowing through the input and the first output of the housing. The first filter also having an oleophobic property to improve a removal of the liquid in case a surface tension of the liquid is lower than a surface tension of bodily fluids due to other components present in the respiratory gas. The housing also having a liquid container to collect the liquid separated by the first filter having both the hydrophobic and oleophobic property.

Various other features, objects, and advantages of the invention will be made apparent to those skilled in art from the accompanying drawings and detailed description thereof.

### BREAF DESCRIPTION OF THE DRAWINGS

Fig. 1 illustrates a setup with a medical gas analyzer connected to the ventilation circuit of a patient.
Fig. 2 shows the principle of one embodiment of a liquid separation apparatus.
Fig. 3 shows the principle of another embodiment of a liquid separation apparatus.
Fig. 4 shows the principle of a third embodiment of a liquid separation apparatus in the gas sampling adapter connected to the respiratory circuit.
Fig. 5 shows a cross section of the adapter in Fig. 4 with the third embodiment of the liquid separation apparatus.
Fig. 6 shows a cross section of an adapter similar to that in Fig. 4, with a fourth embodiment of a liquid separation apparatus.

### DETAILED DESCRIPTION OF THE INVENTION

Specific embodiments are explained in the following detailed description making a reference to accompanying drawings. These detailed embodiments can naturally be modified and should not limit the scope of the invention as set forth in the claims.

A liquid separation apparatus comprising hydrophobic filter upstream an analyzer analyzing gas samples withdrawn from a respiratory gas as described hereinbefore work to satisfaction in the majority of cases but it does not tolerate liquids with a reduced surface tension. Many drugs used in nebulizers include surfactants or the surface tension is reduced for other reasons. Also sputum and blood has a surface tension lower than that of water. The hydrophobic filter must then not only have high enough entry pressure for water but also for liquids with lower surface tension. Such filters are sometimes called superphobic or oleophobic because the surface tension of oil is smaller than that of water. Thus the filter material rejects oil or e.g. alcohol in the same manner as water. By applying in the liquid separation apparatus a filter with oleofobic property problems due to different chemical compositions, which do not belong to normal bodily fluids may be avoided.

As the oleophobic ability of the filter also prevents water entry for instance during the nebulization procedure, the sampling line or adapter does not have to be disconnected during nebulization decreasing patient risks when caregivers do not need to interrupt the monitoring and wait for the adverse medicines to disappear from the respiratory circuit. Continuous gas measurement is one of the key parameters in the intensive care unit environment, also when the patients are nebulized. Also unnecessary module cross-contaminations, breakages and service calls will be eliminated. Normally, patient monitoring devices are cleaned with alcohol based detergents, which may result in these detergents entering the liquid filtering device. A consequent drop in the water entry pressure and wetting of a conventional hydrophobic filter may lead to cross-contamination. A filter with oleophobic property can be made to withstand such detergents and thus prevent unnecessary malfunction and service of the gas analyzer. In addition, the oleophobic treatment will make it hard for bacteria to deposit and reside on the filter surface and therefore the propagation of such micro-organisms is prohibited.

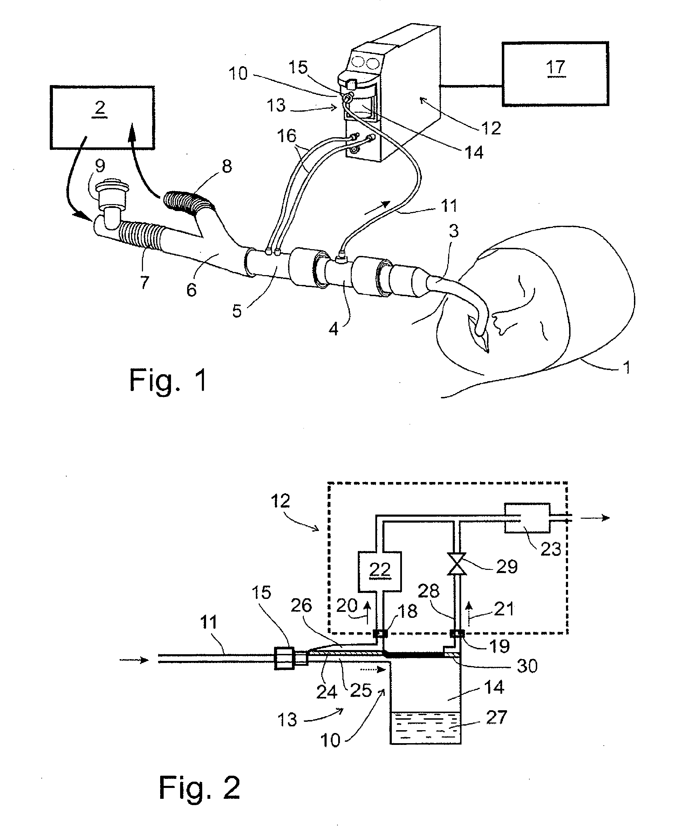

A patient circuit with gas monitoring and nebulization is shown in Figure 1. The patient 1 is connected to a respirator 2 using a respiratory circuit including an intubation tube 3, a gas sampling adapter 4, an optional spirometer sensor 5 and a Y-piece 6, connected to an inspiratory limb 7 and to an expiratory limb 8. In the inspiratory limb 7 there may be connected a nebulizer 9 for nebulizing drugs to the lungs. The gas sampling adapter 4 can be a straight model like the one shown or it can be elbow shaped. A sampling line 11 connects the adapter 4 to the gas analyzer 12 via a liquid separation apparatus 13 with a liquid container 14, also called water trap. The sample flow direction is away from the respiratory circuit into the gas analyzer 12. Normal sampling flow values are between 50 ml/min and 200 ml/min. The spirometer sensor 5, if present, may also be connected to the gas analyzer 12 e.g. using pressure transferring tubing 16. The gas analyzer 12 is connected to a host device 17, which may comprise a display and a control unit. Sometimes the gas analyzer 12 is modular as shown in Figure 1, sometimes it is integrated into the host device 17, both together forming a patient monitor. The nebulizer 9 may be connected to the inspiratory limb 7 of the respiratory circuit but it could also be connected closer to the patient.

The working principle of the liquid separation apparatus 13 and its connections within the gas analyzer 12 is shown in Figure 2. The liquid separation apparatus comprises a housing 10 having an input 15 to deliver a sample withdrawn from the respiratory gas flowing along the respiratory circuit and having a first output 18 for discharging at least part of the sample of the respiratory gas and also having a first filter 24 with a hydrophobic property for removing the liquid from the gas sample. Thus the sampled gas is flowing through the sampling line 11 and the input 15 into the liquid separation apparatus 13, which may be disposable. Also a respiratory gas analyzing system is shown in Figure 2 comprising the liquid separation apparatus 13 referred hereinbefore and a gas analyzer 12 for analyzing respiratory gases. The gas analyzer comprises a gas sensor 22 for measuring at least one gas component and a gas withdrawing apparatus 23 such as a pump for withdrawing respiratory gases to the gas sensor 22. The gas sensor 22 comprises e.g. a non-dispersive IR sensor for detecting carbon dioxide, nitrous oxide and anesthetic agents like halothane, enflurane, isoflurane, desflurane and sevoflurane and it may also comprise an oxygen sensor and other components related to its proper functioning.

The input gas flow through the sampling line 11 is divided into a main flow 20 entering the gas sensor 22 for the gas measurement, and a smaller side flow 21. The main flow 20 enters the gas sensor 22 via a first filter 24, which divides the flow path in a housing 10 into an input channel 25 and an output channel 26. If the sample flow in the sampling tube 11 includes a liquid, normally water, this liquid 27 will end up in the liquid container 14 because the first filter 24 is hydrophobic and, according to the embodiment, also oleophobic to improve a removal of the liquid in case a surface tension of the liquid is lower than a surface tension of bodily fluids such as sputum, blood or mucus. The first filter with both the hydrophobic and oleophobic property lets through only the gas flow 20. The liquid container 14 is kept at negative pressure compared to the measuring side so that its gas content does not disturb the response of the gas sensor 22. This is accomplished by sucking through a second output 19 a small amount of gas as the side flow 21 through a tube 28 and a restrictor 29 directly to the gas withdrawing apparatus 23. The second output is provided with a second filter 30 with both the hydrophobic and oleophobic property being similar to the first filter 24. The second filter 30 is used to prevent the liquid 27 from leaving the liquid container 14 through this second filter with the gas component and from entering the tube 28. This second filter 30 is normally identical to the first filter 24 and can conveniently be the same piece, only separately sealed.

The first and second filters which may be a filter membrane is known in the art to be hydrophobic and it may have a water entry pressure of 0.5 bar or more, meaning that water will not wet the filter and it will penetrate the filter only for pressures in excess of the water entry pressure value. The pore size is normally in the range from 0.2 µm to 5 µm depending on the filtering need for particles, bacteria and virus. Several filter materials like different fluoropolymers and acrylic copolymer are inherently or are possible to make hydrophobic, but the best material is expanded polytetrafluoroethylene (ePTFE), a specially treated fluoropolymer. It can be acquired e.g. from W.L Gore & Associates, Inc., Elkton, MD, USA. The bulk material PTFE has a very low surface energy of 18 mN/m, meaning that only liquids with a surface tension lower than this will spontaneously wet the surface. Most liquids have a surface tension value higher than 18 mN/m so they would never totally wet the surface, but many liquids would still stick to the surface and cause obstruction of the filter. Pure water has a surface tension of 72.8 mN/m and its contact angle on PTFE in equilibrium is about 110 degrees. This is more than the critical angle of 90 degrees and water droplets would not stick to the PTFE surface or wet it. The surface energy of a solid is defined as the work necessary to increase the surface by unit area against the force of surface tension of a liquid. The contact angle is the angle at which a liquid/vapor interface meets a solid surface. For compete wetting the contact angle is close to zero. For a filter there is also a capillary action which influences the transport of liquid through the filter. If the contact angle is more than 90 degrees there would not be any capillary suction into the filter material. As the contact angle is reduced to about or less than 90 degrees the liquid starts to stick to the surface even if it does not spread over the surface. Simultaneously, the capillary suction increases and there is a risk that the liquid eventually will penetrate the filter. As an example, ethanol has a surface tension of 22.1 mN/m at room temperature. On a PTFE surface ethanol will have a contact angle of only about 20 degrees. This means that the capillary suction also is very strong and the ePTFE filter would easily be wetted and penetrated. Once this has happened the filter will also become penetratable for water. The filter recovers only after complete drying.

According to published measurements the surface tension on PTFE resulting in the critical contact angle 90 degrees is about 65 mN/m. This value is very easily reached for contaminated water, e.g. a mixture of 2% ethanol in water has this value. Human saliva is reported to have a surface tension of between 53 and 59 mN/m and also blood at room temperature has a value of 58 mN/m. All surface tension values are reduced at higher temperature. At 37 °C the value for blood is only about 52.6 mN/m. Even if blood is too viscous to pass through the filter it certainly would stick to the surface and block the filter. Different surfactants also reduce the surface tension to make the contact angle much less than 90 degrees. A 4% lidocaine solution for inhalation has e.g. a surface tension of 58.3 mN/m. The liquids, mucus or blood easily stick to the ePTFE hydrophobic filter and block the gas stream to the gas analyzer. The filter is not necessarily penetratable to the water mixtures mentioned but different drugs that are nebulized into the inspiratory limb 7 in Figure 1 could potentially have a fairly low surface tension and thus, if penetrating the filter, could endanger the proper function of the gas analyzer 12 and even the safety of the patient. As an example salbutamol, used to treat asthma and chronic obstructive pulmonary disease (COPD), has as a respirator solution a surface tension of only about 35 mN/m. As mentioned above, it is enough to wet the filter once with a liquid in order to make it penetratable also to water. Therefore, according to the user's manual, the gas analyzer 12 must be disconnected during nebulization.

Fat and oil rejecting surface treatments of PTFE are well known and expanded PTFE filters with a so called oleophobic treatment can be acquired e.g. from GE Energy, Kansas City, MO, USA. Oil repellency may require that the surface energy of the first filter 24 and advantageously also the second filter 30 be lower than 15 mN/m, more specifically lower than 10 mN/m. Even if the main use for these treated filters is for rejecting different oils and fat products the treatment can also be beneficial as an enhanced hydrophobic filter in a liquid separation apparatus 13 according to the embodiments. The treatment normally affects the surface of the material and may be applied as a liquid and then dried and cured to a thin layer. This layer may have a composition e.g. from the fluoroalkyl group. The oleophobic property has been achieved by treating at least one of the opposite surfaces of the first filter 24 and optionally the second filter 30. With surface is generally understood the outer surface of the filter, at least the one possibly in contact with liquid, but also the inner surfaces of the filter structure may be coated. The degree of oil repellency is given as an Oil Repellency Grade Number, from 0 to 8 according to the AATCC Test Method 118-2002 (equivalent to ISO 14419). Grade number 8 has the best performance while 0 fails the test. For rating number 1 the filter must withstand a drop of Kaydol (White mineral oil) for 10 seconds without wetting the surface. For this to happen the contact angle has to be more than 90 degrees, especially if the filter is supposed to withstand a certain entry pressure. Kaydol has a surface tension of 31.5 mN/m and it would soon start to wet a ePTFE filter without any surface treatment. The conventional hydrophobic ePTFE filter has according to the oil repellency test the Oil Repellancy Grade number 0. At the other end of the oil repellency rating number scale the test is performed using n-heptane with a surface tension of 20.0 mN/m. Commercially available ePTFE membranes with oleophobic surface treatment are normally rated between 3 and 8, which would correspond to rejection of liquids with surface tension 27.6 mN/m and 20 mN/m, respectively. Such membranes would also be well suited as filters in a liquid filtering device used to protect a patient gas analyzer. Thus the Oil Repellency Grade number of the first filter 24 and optionally the second filter 30 may be at least 3, more specifically at least 5, even more specifically at least 7. Because liquid repellancy of the filter surface is much increased it means that potential liquids sucked up from the respiratory circuit will not penetrate or even wet the filter and the reduced sticking ensures maximum usable filter surface. No disconnection of the gas sampling system would be needed during nebulization of drugs.

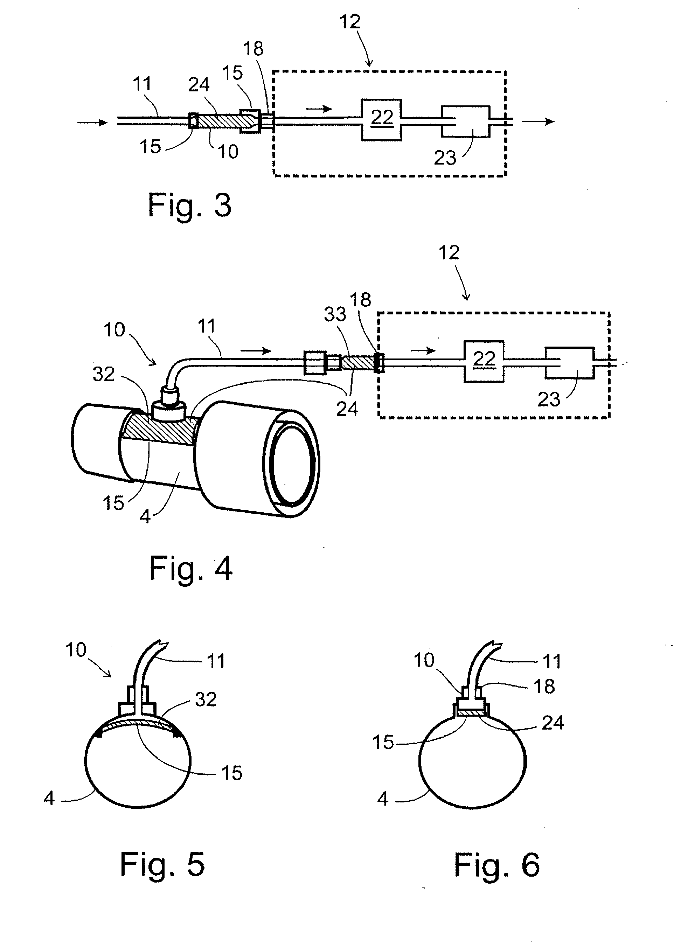

The liquid filtering can also be accomplished according to the principles shown in Figure 3. There is no liquid container in the respiratory gas analyzing system between the sampling line 11 and the gas analyzer 12 or its gas withdrawing apparatus 23, only the first filter 24 with a hydrophobic and oleophobic property between the input 15 and the output 18 of the housing. Water or other liquids cannot be transmitted to the gas sensor 22 because of this first filter 24. Oleophobic properties ensure that no wetting of the filter will occur even during nebulization. A solution like this is applied when large quantities of liquid are not probable. This would be the case for a low sampling flow rate of about 50 ml/min or for use in environments with less probability of water and mucus production, e.g. in neonatal or pediatric departments. The gas analyzer 12 has a simpler construction than that of Figure 2 because of the absent liquid container.

Still another embodiment of a liquid separation apparatus is shown in Figure 4. Now the first filter 24 comprises a protective filter component 33 with one of hydrophobic, oleophobic and hydrophilic swelling property and an oleophobic filter component 32, which are separate components and their properties may be different. The oleophobic filter component 32 and the protective filter component 33 are located between the input 15 and the first output 18 of the housing 10 and which housing may optionally comprise at least part of the gas sampling adapter 4 as shown in Figure 5. This means that no water or mucus can enter the gas sensor 22. The first filter 24 with oleophobic properties is removing the liquid or not allowing the liquid to penetrate this filter in case the surface tension of the liquid is lower than the surface tension of bodily fluids. In that case the first filter will not be wetted by possible nebulization from the nebulizer 9. Regarding the embodiment shown in Figures 4 and 5 there may be reason to enhance the hydrophobic properties of the oleophobic filter component 32 of the first filter 24, because it cannot normally be removed during the procedure due to the patient risks. This embodiment is especially suited for intensive care use. Therefore there is arranged the protective filter component 33 separated from the oleophobic filter component 32 meaning that the protective filter component 33 with one of a hydrophobic, oleophobic and hydrophilic swelling property and the oleophobic filter component 32 with the oleophobic property are arranged in series, in which case the oleophobic filter component 32 is upstream in relation to the protective filter component 33. Both filter components may be located in the housing 10 also comprising in this embodiment the sampling line 11. The protective filter component 33 is upstream the first output 18 guiding the sample flow to the gas analyzer 12 as shown in Figure 4. This protective filter component would prevent condensed water or other liquids from entering the gas analyzer 12 in case of misuse. Protective filter component 33, which may be optional, can be either hydrophobic or oleophobic or it can even be of a hydrophilic swelling type for protection of the first output 18.

In Figure 6 the first filter 24 is at the first input 15, which is also the case in the embodiment shown in Figure 4, but the housing 10 is without at least part of the gas sampling adapter 4 in which case the housing with the first filter 24 can be removed and changed if required to another housing with the new filter during the procedure, but leave the gas sampling adapter in its place allowing the function of the respiratory circuit between the respirator 2 and the patient 1.

The written description uses examples to disclose the invention, including the best mode, and also to enable any person skilled in the art to make and use the invention. The patentable scope of the invention is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal languages of the claims.

## Claims

1. A liquid separation apparatus for removing a liquid from a respiratory gas comprising:
a housing (10) having an input (15) for delivering a sample withdrawn from the respiratory gas,a first output (18) for discharging at least part of the sample of the respiratory gas and a first filter (24) with a hydrophobic property for removing the liquid from the gas sample flowing through said input and said first output of said housing,
**characterized in that** said first filter (24) also having an oleophobic property to improve a removal of the liquid in case a surface tension of the liquid is lower than a surface tension of bodily fluids.

2. The liquid separation apparatus according to claim 1, **characterized in that** said first filter (24) with the oleophobic property has been achieved by treating said first filter with the hydrophobic property.

3. The liquid separation apparatus according to claim 1, **characterized in that** said first filter (24) with the oleophobic property has been achieved by treating at least one of opposite surfaces of said filter with the hydrophobic property.

4. The liquid separation apparatus according to claim 1, **characterized in that** said first filter (24) comprises at least one filter having both the hydrophobic and oleophobic property.

5. The liquid separation apparatus according to claim 1, **characterized in that** said first filter (24) comprises a protective filter component (33) having one of hydrophobic, oleophobic and hydrophilic swelling property and an oleophobic filter component (32) having the oleophobic property.

6. The liquid separation apparatus according to claim 5, **characterized in that** said protective filter component (33) with one of hydrophobic, oleophobic and hydrophilic swelling property and said oleophobic filter component (32) with the oleophobic property are arranged in series, in which case said oleophobic filter component (32) is upstream in relation to said protective filter component (33).

7. The liquid separation apparatus according to claim 1, **characterized in that** said housing (10) also comprising a liquid container (14) to collect the liquid separated by said first filter (24) having both the hydrophobic and oleophobic property.

8. The liquid separation apparatus according to claim 7, **characterized in that** said housing (10) further comprising an input channel (25) receiving the sample of the respiratory gas and an output channel (26) separated by said first filter with both the hydrophobic and oleophobic property and which output channel is adapted to receive at least part of gas components penetrated though said first filter, but the liquid separated by said first filter are guided from said input channel to said liquid container (14).

9. The liquid separation apparatus according to claim 8, **characterized in that** said housing (10) also comprising a second output (19) in flow communication with said liquid container allowing gas withdrawal from said liquid container through said second output, and which second output is provided with a second filter (30) with both the hydrophobic and oleophobic property to prevent the liquid from leaving the liquid container (14) through said second filter with the gas component.

10. The liquid separation apparatus according to claim 1, **characterized in that** said first filter is downstream said input of said housing, but between said input and said output of said housing.

11. The liquid separation apparatus according to claim 1, **characterized in that** a surface energy of said first filter (24) is lower than 15 mN/m, more specifically lower than 10 mN/m.

12. The liquid separation apparatus according to claim 1, **characterized in that** the Oil Repellency Grade number of the first filter (24) is at least 3, more specifically at least 5, even more specifically at least 7.

13. A respiratory gas analyzing system comprising:
a gas analyzer (12) for analyzing respiratory gases, which gas analyzer comprising a gas sensor (22) for measuring at least one gas component and a gas withdrawing apparatus (23) for withdrawing respiratory gases to said gas sensor; and
a liquid separation apparatus (13) upstream said gas sensor comprising a housing (10) having an input (15) for delivering a sample withdrawn, a first output (18) for discharging at least part of the sample of the respiratory gas to said gas sensor and a first filter (24) with a hydrophobic property for removing the liquid from the gas sample flowing through said input of said housing,
**characterized in that** said first filter also having an oleophobic property to improve a removal of the liquid in case a surface tension of the liquid is lower than a surface tension of bodily fluids.

14. The respiratory gas analyzing system according to claim 13 further comprising a gas sampling adapter (4) allowing respiratory gases to flow to and from lungs of the patient and which adapter is in flow communication with said input for delivering the sample of the respiratory gas to said gas analyzer (12).

15. The respiratory gas analyzing system according to claim 14, **characterized in that** said sampling adapter (4) is configured to be in flow communication with a nebulizer (9) nebulising drugs to the respiratory gas and which drugs reduce the surface tension of bodily fluids or other liquids withdrawn.

## Patentansprüche

1. Flüssigkeitsabscheidevorrichtung zum Entfernen einer Flüssigkeit aus einem Atemgas, Folgendes umfassend:
ein Gehäuse (10) mit einem Einlass (15) zum Zuführen einer aus dem Atemgas entnommenen Probe, einen ersten Auslass (18) zum Ablassen mindestens eines Teils der Probe des Atemgases und einen ersten Filter (24) mit einer hydrophoben Eigenschaft zum Entfernen der Flüssigkeit aus der Gasprobe, die durch den Einlass und den ersten Auslass des Gehäuses strömt,
**dadurch gekennzeichnet, dass** der erste Filter (24) außerdem eine oleophobe Eigenschaft aufweist, um ein Entfernen der Flüssigkeit dann zu verbessern, wenn eine Oberflächenspannung der Flüssigkeit geringer als eine Oberflächenspannung von körpereigenen Fluiden ist.

2. Flüssigkeitsabscheidevorrichtung nach Anspruch 1, **dadurch gekennzeichnet, dass** der erste Filter (24) mit der oleophoben Eigenschaft durch Behandeln des ersten Filters mit der hydrophoben Eigenschaft erzielt wurde.

3. Flüssigkeitsabscheidevorrichtung nach Anspruch 1, **dadurch gekennzeichnet, dass** der erste Filter (24) mit der oleophoben Eigenschaft durch Behandeln mindestens einer von gegenüberliegenden Oberflächen des Filters mit der hydrophoben Eigenschaft erzielt wurde.

4. Flüssigkeitsabscheidevorrichtung nach Anspruch 1, **dadurch gekennzeichnet, dass** der erste Filter (24) mindestens einen Filter umfasst, der sowohl die hydrophobe als auch die oleophobe Eigenschaft aufweist.

5. Flüssigkeitsabscheidevorrichtung nach Anspruch 1, **dadurch gekennzeichnet, dass** der erste Filter (24) eine Schutzfilterkomponente (33) umfasst, die eine hydrophobe oder eine oleophobe Eigenschaft oder eine hydrophile Quelleigenschaft aufweist, und eine oleophobe Filterkomponente (32), welche die oleophobe Eigenschaft aufweist.

6. Flüssigkeitsabscheidevorrichtung nach Anspruch 5, **dadurch gekennzeichnet, dass** die Schutzfilterkomponente (33) mit der hydrophoben oder der oleophoben Eigenschaft oder der hydrophilen Quelleigenschaft und die oleophobe Filterkomponente (32) mit der oleophoben Eigenschaft in Reihe angeordnet sind, wobei sich die oleophobe Filterkomponente (32) in prozessaufwärtigem Verhältnis zur Schutzfilterkomponente (33) befindet.

7. Flüssigkeitsabscheidevorrichtung nach Anspruch 1, **dadurch gekennzeichnet, dass** das Gehäuse (10) ferner einen Flüssigkeitsbehälter (14) umfasst, um die Flüssigkeit zu sammeln, die vom ersten Filter (24) mit sowohl der hydrophoben als auch der oleophoben Eigenschaft abgeschieden wurde.

8. Flüssigkeitsabscheidevorrichtung nach Anspruch 7, **dadurch gekennzeichnet, dass** das Gehäuse (10) ferner einen Einlasskanal (25) umfasst, der die Probe des Atemgases aufnimmt, und einen Auslasskanal (26), der durch den ersten Filter mit sowohl der hydrophoben als auch der oleophoben Eigenschaft geteilt ist, wobei der Auslasskanal dafür eingerichtet ist, mindestens einen Teil der Gaskomponenten aufzunehmen, die durch den ersten Filter gedrungen sind, die vom ersten Filter abgeschiedene Flüssigkeit aber vom Einlasskanal zum Flüssigkeitsbehälter (14) geführt wird.

9. Flüssigkeitsabscheidevorrichtung nach Anspruch 8, **dadurch gekennzeichnet, dass** das Gehäuse (10) außerdem einen zweiten Auslass (19) in Strömungsverbindung mit dem Flüssigkeitsbehälter umfasst, was einen Gasabzug aus dem Flüssigkeitsbehälter durch den zweiten Auslass ermöglicht, wobei der zweite Auslass mit einem zweiten Filter (30) mit sowohl der hydrophoben als auch der oleophoben Eigenschaft ausgestattet ist, um zu verhindern, dass die Flüssigkeit durch den zweiten Filter mit der Gaskomponente aus dem Flüssigkeitsbehälter (14) austritt.

10. Flüssigkeitsabscheidevorrichtung nach Anspruch 1, **dadurch gekennzeichnet, dass** der erste Filter prozessabwärts des Einlasses des Gehäuses, aber zwischen dem Einlass und dem Auslass des Gehäuses liegt.

11. Flüssigkeitsabscheidevorrichtung nach Anspruch 1, **dadurch gekennzeichnet, dass** eine Oberflächenenergie des ersten Filters (24) geringer als 15 mN/m ist, insbesondere geringer als 10 mN/m.

12. Flüssigkeitsabscheidevorrichtung nach Anspruch 1, **dadurch gekennzeichnet, dass** das Ölabweisungsvermögen des ersten Filters (24) mindestens Klasse 3, insbesondere mindestens Klasse 5 und noch spezieller mindestens Klasse 7 ist.

13. Atemgas-Analysesystem, Folgendes umfassend:
einen Gasanalysator (12) zum Analysieren des Atemgases, wobei der Gasanalysator einen Gassensor (22) zum Messen mindestens einer Gaskomponente und eine Gasabzugsvorrichtung (23) zum Abziehen von Atemgasen zu dem Gassensor umfasst, und
eine Flüssigkeitsabscheidevorrichtung (13) prozessaufwärts des Gassensors, die ein Gehäuse (10) mit einem Einlass (15) zum Zuführen einer entnommenen Probe, einen ersten Auslass (18) zum Ablassen mindestens eines Teils der Probe des Atemgases zum Gassensor und einen ersten Filter (24) mit einer hydrophoben Eigenschaft zum Entfernen der Flüssigkeit aus der Gasprobe umfasst, die durch den Einlass des Gehäuses strömt,
**dadurch gekennzeichnet, dass** der erste Filter außerdem eine oleophobe Eigenschaft aufweist, um ein Entfernen der Flüssigkeit dann zu verbessern, wenn eine Oberflächenspannung der Flüssigkeit geringer als eine Oberflächenspannung von körpereigenen Fluiden ist.

14. Atemgas-Analysesystem nach Anspruch 13, ferner einen Gasentnahmeadapter (4) umfassend, der das Strömen von Atemgasen zu und von den Lungen des Patienten ermöglicht, und wobei der Adapter in Strömungsverbindung mit dem Einlass zum Zuführen der Atemgasprobe zum Gasanalysator (12) steht.

15. Atemgas-Analysesystem nach Anspruch 14, **dadurch gekennzeichnet, dass** der Entnahmeadapter (4) dafür gestaltet ist, in Strömungsverbindung mit einem Zerstäuber (9) zu stehen, der Arzneimittel in das Atemgas zerstäubt, wobei die Arzneimittel die Oberflächenspannung körpereigener Fluide oder anderer abgezogener Flüssigkeiten verringern.

## Revendications

1. Appareil de séparation de liquides pour éliminer un liquide d'un gaz respiratoire, comprenant :
un boîtier (10) ayant une entrée (15) pour délivrer un échantillon retiré du gaz respiratoire, une première sortie (18) pour décharger au moins une partie de l'échantillon du gaz respiratoire et un premier filtre (24) à propriété hydrophobe pour éliminer le liquide de l'échantillon de gaz s'écoulant à travers ladite entrée et ladite première sortie du boîtier,
**caractérisé en ce que** ledit premier filtre (24) présente également une propriété oléophobe pour améliorer l'élimination du liquide dans le cas où la tension superficielle du liquide est inférieure à la tension superficielle des fluides corporels.

2. Appareil de séparation de liquides selon la revendication 1, **caractérisé en ce que** ledit premier filtre (24) à propriété oléophobe a été obtenu en traitant ledit premier filtre à propriété hydrophobe.

3. Appareil de séparation de liquides selon la revendication 1, **caractérisé en ce que** ledit premier filtre (24) à propriété oléophobe a été obtenu en traitant au moins l'une des surfaces opposées dudit filtre à propriété hydrophobe.

4. Appareil de séparation de liquides selon la revendication 1, **caractérisé en ce que** ledit premier filtre (24) comprend au moins un filtre ayant à la fois les propriétés hydrophobe et oléophobe.

5. Appareil de séparation de liquides selon la revendication 1, **caractérisé en ce que** ledit premier filtre (24) comprend un composant de filtre protecteur (33) ayant l'une des propriétés hydrophobe, oléophobe et hydrophile gonflante et un composant de filtre oléophobe (32) à propriété oléophobe.

6. Appareil de séparation de liquides selon la revendication 5, **caractérisé en ce que** ledit composant de filtre protecteur (33) avec l'une des propriétés hydrophobe, oléophobe et hydrophile gonflante et ledit composant de filtre oléophobe (32) à propriété oléophobe sont aménagés en série, auquel cas ledit composant de filtre oléophobe (32) se trouve en amont par rapport audit composant de filtre protecteur (33).

7. Appareil de séparation de liquides selon la revendication 1, **caractérisé en ce que** ledit boîtier (10) comprend également un récipient de liquide (14) pour recueillir le liquide séparé par ledit premier filtre (24) à propriétés à la fois hydrophobe et oléophobe.

8. Appareil de séparation de liquides selon la revendication 7, **caractérisé en ce que** ledit boîtier (10) comprend en outre un canal d'entrée (25) recevant l'échantillon du gaz respiratoire et un canal de sortie (26) séparé par ledit premier filtre à propriétés à la fois hydrophobe et oléophobe, lequel canal de sortie est à même de recevoir au moins une partie de composants gazeux qui sont passés travers ledit premier filtre, mais le liquide séparé par ledit premier filtre est guidé dudit premier canal d'entrée audit récipient de liquide (14).

9. Appareil de séparation de liquides selon la revendication 8, **caractérisé en ce que** ledit boîtier (10) comprend également une seconde sortie (19) en communication fluidique avec ledit récipient de liquide permettant le retrait du gaz dudit récipient de liquide à travers ladite seconde sortie, la seconde sortie étant pourvue d'un second filtre (30) à propriétés à la fois hydrophobe et oléophobe pour empêcher le liquide de quitter le récipient de liquide (14) à travers ledit second filtre avec le composant gazeux.

10. Appareil de séparation de liquides selon la revendication 1, **caractérisé en ce que** ledit premier filtre est situé en aval de ladite entrée dudit boîtier, mais entre ladite entrée et ladite sortie dudit boîtier.

11. Appareil de séparation de liquides selon la revendication 1, **caractérisé en ce que** l'énergie de surface dudit premier filtre (24) est inférieure à 15 mN/m, plus spécifiquement inférieure à 10 mN/m.

12. Appareil de séparation de liquides selon la revendication 1, **caractérisé en ce que** l'indice de qualité de répulsion à l'huile du premier filtre (24) est d'au moins 3, plus spécifiquement d'au moins 5, encore plus spécifiquement d'au moins 7.

13. Système d'analyse de gaz respiratoire comprenant :
un analyseur de gaz (12) pour analyser des gaz respiratoires, ledit analyseur de gaz comprenant un capteur de gaz (22) pour mesurer au moins un composant de gaz et un appareil de retrait de gaz (23) pour retirer des gaz respiratoires vers ledit capteur de gaz ; et
un appareil de séparation de liquides (13) situé en amont dudit capteur de gaz et comprenant un boîtier (10) ayant une entrée (15) pour délivrer un échantillon retiré, une première sortie (18) pour décharger au moins une partie de l'échantillon du gaz respiratoire vers ledit capteur de gaz et un premier filtre (24) à propriété hydrophobe pour retirer le liquide de l'échantillon de gaz s'écoulant à travers ladite entrée dudit boîtier,
**caractérisé en ce que** ledit premier filtre présente également un propriété oléophobe pour améliorer le retrait du liquide dans le cas où la tension superficielle du liquide est inférieure à la tension superficielle des fluides corporels.

14. Système d'analyse de gaz respiratoire selon la revendication 13, comprenant en outre un adaptateur d'échantillonnage de gaz (4) permettant aux gaz respiratoires de s'écouler dans les poumons du patient et dans l'autre sens, lequel adaptateur est en communication fluidique avec ladite entrée pour délivrer l'échantillon de gaz respiratoire audit analyseur de gaz (12).

15. Système d'analyse de gaz respiratoire selon la revendication 14, **caractérisé en ce que** ledit adaptateur d'échantillonnage (4) est configuré pour être en communication fluidique avec un nébuliseur (9) qui nébulise des médicaments dans le gaz respiratoire, lesquels médicaments réduisent la tension superficielle des fluides corporels ou d'autres liquides retirés.
